# EUROPÄISCHE PATENTANMELDUNG

(11) **EP 3 153 856 A1**
(43) Veröffentlichungstag der Anmeldung: **12.04.2017**
(21) Anmeldenummer: 15188536.5
(22) Anmeldetag: 06.10.2015
(51) Int. Cl.: G01N 33/49, G01N 15/05, G01N 33/569, G01N 33/80, G01N 15/00

(54) **VERFAHREN ZUR BESTIMMUNG DER KONZENTRATION VON RETIKULIERTEN THROMBOZYTEN**

(71) Anmelder: Siemens Healthcare Diagnostics Inc., Tarrytown, NY 10591-5098 (US); Siemens Healthcare Diagnostics GmbH, 1210 Wien (AT)
(72) Erfinder: Prechtl, Gottfried, 2051 Zellnerndorf (AT); Jones, Val, Malahide Co Dublin (IE)
(74) Vertreter: Maier, Daniel Oliver

(57) **Zusammenfassung**

Die Erfindung betrifft ein Verfahren zur Bestimmung der Konzentration von retikulierten Thrombozyten in einer Thrombozyten enthaltenden Probe (4) eines Biofluids, insbesondere in einer Blutprobe, wobei der Probe (4) des Biofluids ein für Nukleinsäure selektiver Farbstoff zugeführt wird, wobei nach der Anfärbung in der Probe (4) im Absorptionsbereich des Farbstoffes eine Vielzahl von optischen, partikelbedingten Messwerten jeweils als Tupel aus Brechungsindex und Absorptionsgrad bestimmt wird, wobei Messwerte mit einem Absorptionsgrad innerhalb eines Teilbereiches (6) aller gemessenen Werte des Absorptionsgrades und mit einem Brechungsindex innerhalb eines Teilbereiches (7) aller gemessenen Werte des Brechungsindexes selektiert werden, und wobei die Anzahl der selektierten Messwerte zur Bestimmung der Konzentration der retikulierten Thrombozyten herangezogen wird.

## Beschreibung

Die Erfindung betrifft ein Verfahren zu einer insbesondere vollautomatisierten Bestimmung der Konzentration von retikulierten Thrombozyten in einer Thrombozyten enthaltenden Probe eines Biofluids, insbesondere in einer Blutprobe. Die Erfindung betrifft weiter ein Analysesystem zu einer insbesondere vollautomatisierten Durchführung dieses Verfahrens.

Retikulierte Thrombozyten sind unreife Thrombozyten, die durch Abschnürung aus den Megakaryozyten gebildet werden. Wie Retikulozyten, also unreife Erythrozyten, liefern retikulierte Thrombozyten einen Hinweis auf den zugrundeliegenden Bildungsprozess, vorliegend die Thrombopoese, wobei die Anzahl der retikulierten Thrombozyten mit der Aktivität der Megakaryozyten korreliert. Eine Bestimmung der Konzentration, also einer relativen Anzahl (Fraktion) oder absoluten Anzahl an retikulierten Thrombozyten, gibt die Möglichkeit, Krankheiten zu diagnostizieren, die Einfluss auf die Aktivität der Megakaryozyten nehmen oder die als Krankheitsfolge einen Verbrauch oder eine Destruktion von Thrombozyten zur Folge haben. Beispiele sind hierbei ITP (Immunthrombozytopenie), TTP (Thrombotisch-thrombozytopenische Purpura), DIC (disseminierte intravasale Koagulopathie), HIT (Heparininduzierte Thrombozytopenie), HUS (hämolytisch-urämisches Syndrom) und andere.

Der Prozentsatz an retikulierten Thrombozyten zur gesamten Anzahl an Thrombozyten kann insbesondere mithelfen, eine entsprechende Diagnose bei Thrombozythopenie zu erstellen. Die Analyse dieses Wertes in Proben von Biofluiden, insbesondere in Blutproben, soll möglichst vollautomatisiert erfolgen. Gegenwärtig ist dies mit automatisch arbeitenden optischen Analysesystemen dadurch ermöglicht, dass die in retikulierten Thromobzyten noch vermehrt enthaltene RNA (Ribonukleinsäure)mit einem Farbstoff angefärbt und fluoreszenzoptisch die Konzentration an enthaltenen retikulierten Thrombozyten bzw. deren absolute Anzahl oder Fraktion ermittelt wird.

Aufgabe der Erfindung ist es, ein alternatives Verfahren zur Bestimmung der Konzentration an retikulierten Thrombozyten in Proben eines Biofluids anzugeben, welches sich ebenfalls automatisiert mit optischen Analysesystemen durchführen lässt.

Diese Aufgabe wird erfindungsgemäß dadurch gelöst, dass einer Probe des Biofluids ein für Nukleinsäure selektiver Farbstoff zugeführt wird, der hier RNA einfärbt, wobei nach der Anfärbung in der Probe im Absorptionsbereich des Farbstoffes eine Vielzahl von optischen, partikelbedingten Messwerten jeweils als Tupel aus Brechungsindex und Adsorptionsgrad bestimmt wird, wobei Messwerte mit einem Adsorptionsgrad innerhalb eines Teilbereiches aller gemessenen Werte des Adsorptionsgrades und mit einem Brechungsindex innerhalb eines Teilbereiches aller gemessenen Werte des Brechungsindexes selektiert werden, und wobei die Anzahl der selektierten Messwerte zur Bestimmung der Konzentration der retikulierten Thrombozyten herangezogen wird.

Die Erfindung geht dabei von der Überlegung aus, dass sich mit einem zytometrischen Verfahren, welches beispielsweise in Durchflussgeometrie durchgeführt wird, bei einer Gegenüberstellung der zweidimensionalen Messwerte aus Absorptionsgrad und zugehörigem Brechungsindex in einem Zytogramm die retikulierten Thrombozyten nicht nur vergleichsweise einfach von reifen Thrombozyten, sondern beispielsweise auch von Erythrozyten getrennt werden können. Da retikulierte Thrombozyten noch relativ viel RNA enthalten, unterscheidet sich nach selektiver Anfärbung deren Absorptionsgrad zunächst von dem reifer Thrombozyten, die wenig RNA erhalten. Insofern kann in einem Zytogramm aus Absorptionsgrad und Brechungsindex ein Teilbereich mit höherem Absorptionsgrad den von retikulierten Thrombozyten stammenden Messwerten zugeordnet werden. Andererseits zeigen Erythrozyten aufgrund ihres Hämoglobingehalts gegenüber Thrombozyten einen höheren Brechungsindex, so dass ein Teilbereich niedrigeren Brechungsindexes den von Thrombozyten stammenden Messwerten zugeordnet werden kann. Insofern kann aus einer zytometrischen Erfassung aus Absorptionsgrad und Brechungsindex ein Teilbereich an Messwerten den retikulierten Thrombozyten zugeordnet werden. Die Anzahl der entsprechend selektierten Messwerte wird dann zur Bestimmung der Konzentration der retikulierten Thrombozyten herangezogen. Es hat sich dabei gezeigt, dass die Messung gegenüber Erythrozyten weitgehend interferenzfrei ist.

Eine insbesondere zweidimensionale zytometrische Messung von optischen partikelrelevanten Merkmalen gehört zum Standardverfahren von automatisierten optischen Analysesystemen von Biofluiden. Jedoch wird die Anfärbung von RNA mittels eines Farbstoffs bislang hauptsächlich zur Bestimmung der Anzahl oder der Fraktion an Retikulozyten durchgeführt. Die Erfindung unterscheidet sich jedoch hiervon.

Die Erfindung ermöglicht weiter auch eine weitgehend interferenzfreie Erfassung der retikulierten Thrombozyten gegenüber Fragmenten von Leukozyten und anderen Teilchen, die einen vergleichbaren Absorptionsgrad wie die retikulierten Thrombozyten aufweisen. Es hat sich gezeigt, dass derartige Fragmente und Teilchen gegenüber den retikulierten Thrombozyten einen geringeren Brechungsindex zeigen.

Zum Ermitteln der Messwerte, die jeweils ein Tupel aus Absorptionsgrad und Brechungsindex umfassen, wird beispielsweise in Durchflussgeometrie zeitaufgelöst die jeweils durch im Messvolumen befindliche Partikel verursachte Streuung, Reflexion und/oder Transmisson eines Lichtstrahls, insbesondere eines Laserstrahls, erfasst. Aus den in verschiedenen Messgeometrien gemessenen optischen Parametern werden dann als Messwerte jeweils Tupel aus optischen Merkmalen, insbesondere auch Brechungsindex und Absorptionsgrad, ermittelt, die Rückschlüsse auf die als disperse Phase in dem Biofluid enthaltenen Partikeln erlauben. Diese sogenannte Durchflusszytometrie ist hierbei ein gängiges Messverfahren moderner optischer Analysesysteme zur Analyse von Biofluiden, wie sie beispielsweise zu einer vollautomatisierten Erstellung eines Blutbildes oder dergleichen eingesetzt werden. Bevorzugt wird daher das vorbeschriebene Verfahren, insbesondere vollautomatisiert, in einem Analysesystem zur optischen Analyse einer Probe eines Biofluids durchgeführt. Als ein geeigneter Farbstoff wird bevorzugt Oxazin 750 verwendet. Oxazin 750 wird häufig zur Anfärbung von Nukleinsäure und vorliegend zur Anfärbung von RNA eingesetzt.

In einer vorteilhaften Ausgestaltung wird die obere Grenze des Teilbereiches des Brechungsindexes, insbesondere veränderlich über den Absorptionsgrad, derart gewählt, dass Brechungsindizes der Erythrozyten von Brechungsindizes der Thrombozyten separiert werden, sodass eine Interferenz ausgeschlossen oder zumindest minimiert wird. Gewöhnlich liegen die Brechungsindizes von Erythrozyten und Thrombozyten weit auseinander, so dass eine entsprechende Grenze leicht durch die Betrachtung von entsprechenden Brechungsindizes festgelegt werden kann. Derartige Brechungsindizes können bei automatisierten Analysesystemen aus entsprechenden Ausgabekanälen entnommen werden.

Hypochrome Erythrozyten, also Erythrozyten mit einem vergleichsweise geringen Hämoglobingehalt, können jedoch einen Brechungsindex zeigen, der in der Nähe der Brechungsindizes von Thrombozyten liegt. Aus diesem Grund wird in einer vorteilhaften Variante die Häufigkeitsverteilung der gemessenen Brechungsindizes ermittelt und hierin eine Population der Erythrozyten in einem Bereich höherer Brechungsindizes und eine Population der Thrombozyten in einem Bereich niedrigerer Brechungsindizes identifiziert. Als obere Grenze des Teilbereiches des Brechungsindizes wird dann ein Brechungsindex aus einem Bereich zwischen den beiden Populationen gewählt. Auf diese Weise wird sichergestellt, dass eine mögliche Interferenz von Messwerten von Thrombozyten und Messwerten von Erythrozyten weitestgehend vermieden ist.

Weiter bevorzugt wird die untere Grenze des Teilbereiches des Absorptionsgrades derart gewählt, dass Absorptionsgrade reifer Thrombozyten von Absorptionsgraden retikulierter Thrombozyten separiert werden. Dies ist deswegen möglich, da wie bereits erwähnt reife Thrombozyten keine oder nur noch einen sehr geringen Anteil an RNA aufweisen, so dass deren Absorptionsgrad in Bezug auf den Absorptionsbereich des eingesetzten Farbstoffes gegenüber dem von retikulierten Thrombozyten verringert ist.

Es hat sich gezeigt, dass die Absorption von Thrombozyten patientenspezifisch ist und somit von Probe zu Probe variiert. Zweckmäßigerweise werden daher in der jeweiligen Probe des Biofluids die Absorptionsgrade der Thrombozyten gemessen und deren Häufigkeitsverteilung ermittelt. Aus der Häufigkeitsverteilung wird ein Häufigkeitsmaximum bestimmt und als untere Grenze des Teilbereiches des Absorptionsgrades wird ein Absorptionsgrad oberhalb des dem Häufigkeitsmaximum zugeordneten Absorptionsgrad gewählt. Auf diese Weise wird eine konkrete spezifische Grenze im Absorptionsgrad bestimmt, die für die jeweilige Probe die reifen Thrombozyten von unreifen, retikulierten Thrombozyten trennt.

Vorteilhaft wird hierbei der Abstand der unteren Grenze des Teilbereiches des Absorptionsgrades zu dem dem Häufigkeitsmaximum zugeordneten Absorptionsgrad derart gewählt, dass die für ein Normbiofluid, z.B. eine Blutprobe eines gesunden Patienten, aus den selektierten Messwerten ermittelte Fraktion der retikulierten Thrombozyten bezüglich aller Thrombozyten in einem Bereich zwischen 1 % und 7 % liegt. Der normale Durchschnittswert liegt hierbei bei 3 %.

Als Wahl dieser Grenze im Absorptionsgrad liegt die Überlegung zugrunde, dass die Lebensdauer an menschlichen Thrombozyten in peripherem Blut etwa 10 Tage beträgt. Daher beträgt die tägliche Reproduktionsrate der Thrombozyten etwa 10 %, um die Thrombozytenzahl konstant zu halten. Wenn man davon ausgeht, dass die Reifungszeit der Thrombozyten in der Peripherie 6 bis 8 Stunden beträgt, liegen somit Durchschnittswerte an retikulierten Thrombozyten für gesunde Normalpatienten bei etwa 3 %.

Zweckmäßigerweise wird die untere Grenze des Teilbereiches des Brechungsindexes, insbesondere veränderlich über den Absorptionsgrad, derart gewählt, dass Brechungsindizes der Thrombozyten von Brechungsindizes von Fragmenten der Leukozyten und anderen Teilchen separiert werden. Wie bereits ausgeführt ist dies möglich, da Fragmente von Leukozyten und auch andere Teilchen, die einen den Thrombozyten vergleichbaren Absorptionsgrad zeigen, einen verringerten Brechungsindex aufweisen.

In einer weiteren bevorzugten Ausgestaltung des Verfahrens wird in der Probe des Biofluids, insbesondere automatisiert, eine Anzahl von Thrombozyten ermittelt, wobei aus der Anzahl der selektierten Messwerte und der Anzahl der Thrombozyten eine Fraktion der retikulierten Thrombozyten ermittelt wird. Bei dieser Variante wird die Gesamtzahl an Thrombozyten separat unabhängig vom Zytogramm aus Absorptionsgrad und Brechungsindex vermessen bzw. aus einem entsprechenden separaten Ausgabekanal eines automatisierten Analysesystems entnommen. Die Anzahl der selektierten Messwerte wird dann in Relation zu dieser separat ermittelten Gesamtzahl der Thrombozyten gesetzt. Bei dieser Variante wird davon ausgegangen, dass die Gesamtzahl an Thrombozyten nicht dem gleichen Zytogramm entnommen werden kann, aus dem die Anzahl an retikulierten Thrombozyten ermittelt wird, da der Brechungsindex der Thrombozyten durch die angefärbte RNA verändert wird.

Die Erfindung betrifft weiter ein Analysesystem zur optischen Analyse einer Probe eines Biofluids, insbesondere einer Blutprobe, welches eingerichtet und ausgebildet ist, ein Verfahren der vorbeschriebenen Art, insbesondere vollautomatisiert, durchzuführen.

Ausführungsbeispiele der Erfindung werden anhand einer Zeichnung näher erläutert. Dabei zeigen:
- FIG 1: schematisch ein automatisiertes optisches Analysesystem für Proben eines Biofluids,
- FIG 2: ein Zytogramm, wobei der Brechungsindex "Refractive Index" gegenüber dem Absorptionsgrad "Absorption" aufgetragen ist,
- FIG 3: ein Histogramm gemessener Brechungsindizes,
- FIG 4: ein weiteres Zytogramm, wobei der Brechungsindex über dem Absorptionsgrad aufgetragen ist,
- FIG 5: ein Histogramm der für Thrombozyten gemessenen Absorptionsgrade und
- FIG 6: in einer Gegenüberstellung verschiedene Zytogramm mit unterschiedlichen Grenzen zwischen reifen und unreifen Thrombozyten.

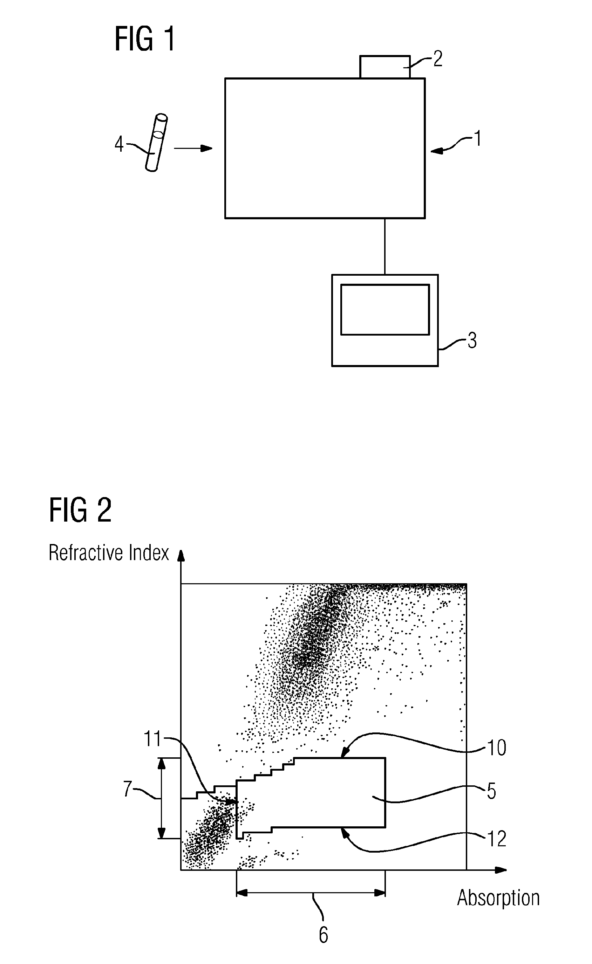

In FIG 1 ist schematisch ein optisches Analysesystem 1 zur Durchführung einer automatisierten Analyse der Probe eines Biofluids dargestellt. Das Analysesystem 1 umfasst hierbei eine Lichtquelle 2, insbesondere einen Laser, der ein Probenvolumen durchstrahlt. Eine Vielzahl von optischen Merkmalen, wie insbesondere partikelbezogene Brechungsindizes, Absorptionsgrade, Transmissionsgrade, Reflexionsgrade etc. werden durch Beobachtung des ein Probenvolumen durcheilenden Laserlichts in Streu-, Reflexions-, Transmissionsgeometrie etc. erfasst. Weiter ist dem Analysesystem 1 eine Ausgabeeinheit 3 zugeordnet. Über die Ausgabeeinheit 3 können über eine Vielzahl von Ausgabekanälen die jeweils ermittelten optischen Merkmale ausgegeben, dargestellt oder weiter ausgewertet werden. Über die Ausgabeeinheit 3 ist es insbesondere möglich, ein vollständiges Blutbild in tabellarischer Form mit Ausgabe aller relevanter Daten einschließlich der Fraktion an retikulierten Thrombozyten auszugeben. Zur Analyse wird eine Probe 4 eines Biofluids, insbesondere eine Blutprobe, dem Analysesystem 1 entsprechend zugeführt. Die anschließende Auswertung geschieht vollautomatisch einschließlich einer etwaigen Zuführung von Hilfs-, Färbestoffen usw.

In FIG 2 ist ein Zytogramm dargestellt, in dem der Brechungsindex gegenüber dem Absorptionsgrad aufgetragen ist. Die erfassten partikelbezogenen Messwerte, die jeweils ein Tupel aus Brechungsindex und Absorptionsgrad umfassen, sind als Ergebnis eines automatisierten Analyseverfahrens eingetragen. Aus einem Bereich 5 selektiver Messwerte kann die Anzahl an retikulierten Thrombozyten entnommen werden. Dieser Bereich umfasst einen Teilbereich 6 der gemessenen Absorptionsgrade und einen Teilbereich 7 der gemessenen Brechungsindizes. Der Bereich 5 ist hierbei insbesondere gegenüber den Messwerten von Erythrozyten durch eine obere Grenze 10 des Brechungsindexes, gegenüber reifen Thrombozyten durch eine untere Grenze 11 des Absorptionsgrades und gegenüber Fragmenten von Leukozyten und anderen Teilchen durch eine untere Grenze 12 des Brechungsindexes abgetrennt. Über die eingezeichneten Grenzen kann die Anzahl der retikulierten Thrombozyten weitestgehend interferenzfrei bestimmt werden.

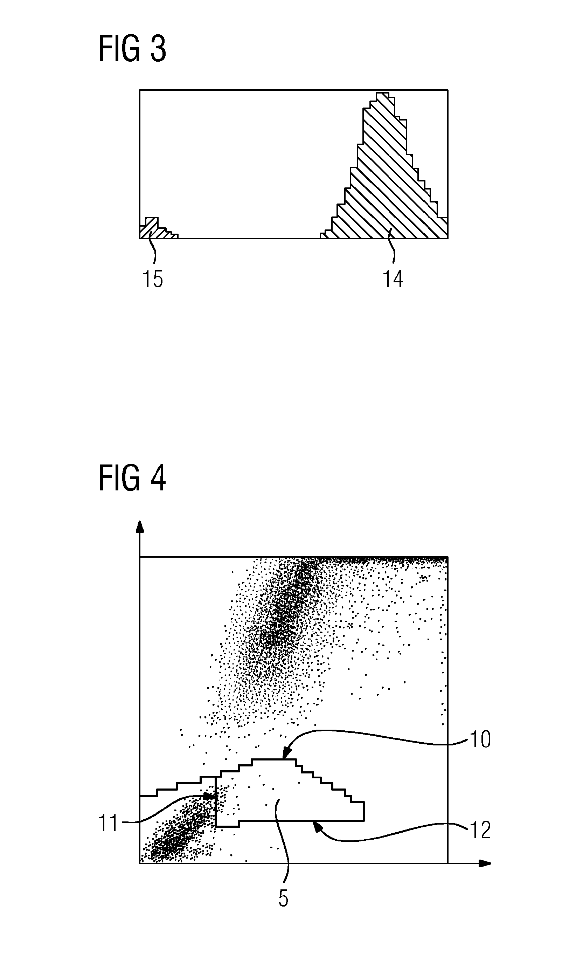

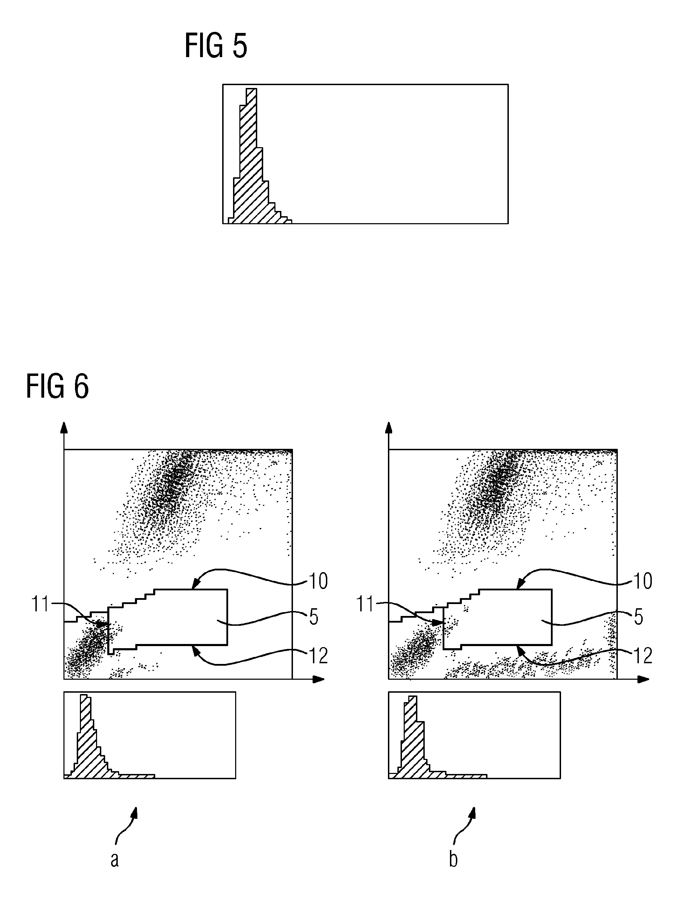

Die obere Grenze 10 des Brechungsindexes zur Abgrenzung der Messwerte von retikulierten Thrombozyten gegenüber Messwerten von Erythrozyten wird aus einem Histogramm gemäß FIG 3, in dem die Häufigkeiten der jeweils gemessenen Brechungsindizes dargestellt ist, ermittelt. Es wird hierbei die große Population 14 an Erythrozyten und die kleine Population 15 an Thrombozyten identifiziert. Zwischen den beiden Populationen 14, 15 wird die obere Grenze eines Brechungsindexes zur Ermittlung der retikulierten Thrombozyten festgelegt. Die entsprechende obere Grenze 10 des Brechungsindexes ist in FIG 4 dargestellt, die ein anderes Zytogramm ähnlich FIG 2 zeigt. Zur Bestimmung der unteren Grenze des Absorptionsgrades für den Bereich 5 der retikulierten Thrombozyten werden gemäß FIG 5 in einem Histogramm die Häufigkeiten der in der Probe des Biofluids gemessenen Absorptionsgrade der Thrombozyten (anderer Kanal) ermittelt. Es wird hierin der jeweilige dem Maximum zugeordnete Absorptionsgrad bestimmt. In den Zytogrammen gemäß FIG 6 werden als untere Grenzen 11 des jeweiligen Absorptionsgrades Absorptionsgrade größer als der dem Maximum zugeordnete Absorptionsgrad festgelegt. In FIG 6 (a) ist die untere Grenze 11 des Absorptionsgrades gegenüber der unteren Grenze 11 des Absorptionsgrades in FIG 6 (b) zu kleineren Werten verschoben.

Durch die vorbeschriebene Bestimmung der unteren Grenze 11 des Absorptionsgrades wird spezifisch auf die Tatsache Bezug genommen, dass Thrombozyten verschiedener Patienten unterschiedliche Absorptionsgrade zeigen. Vorliegend hat es sich gezeigt, dass die untere Grenze des Absorptionsgrades gegenüber dem dem Maximum zugehörenden Absorptionsgrad um eine Anzahl von Schritten bzw. entsprechenden Kanälen nach rechts zu verschieben ist, somit also der Absorptionsgrad der unteren Grenze gegenüber dem Absorptionsgrad am Maximum der Häufigkeitsverteilung größer gewählt ist.

### Bezugszeichenliste

- 1: Analysesystem
- 2: Lichtquelle
- 3: Ausgabegerät
- 4: Probe Biofluid (Blutprobe)
- 5: Bereich selektierter Messwerte
- 6: Teilbereich Absorptionsgrad
- 7: Teilbereich Brechungsindex
- 10: obere Grenze Teilbereich Brechungsindex
- 11: untere Grenze Teilbereich Absorptionsgrad
- 12: untere Grenze Teilbereich Brechungsindex
- 14: Population Erythrozyten
- 15: Population Thrombozyten

## Patentansprüche

1. Verfahren zur Bestimmung der Konzentration von retikulierten Thrombozyten in einer Thrombozyten enthaltenden Probe (4) eines Biofluids, insbesondere in einer Blutprobe, wobei der Probe (4) des Biofluids ein für Nukleinsäure selektiver Farbstoff zugeführt wird, wobei nach der Anfärbung in der Probe (4) im Absorptionsbereich des Farbstoffes eine Vielzahl von optischen, partikelbedingten Messwerten jeweils als Tupel aus Brechungsindex und Absorptionsgrad bestimmt wird, wobei Messwerte mit einem Absorptionsgrad innerhalb eines Teilbereiches (6) aller gemessenen Werte des Absorptionsgrades und mit einem Brechungsindex innerhalb eines Teilbereiches (7) aller gemessenen Werte des Brechungsindexes selektiert werden, und wobei die Anzahl der selektierten Messwerte zur Bestimmung der Konzentration der retikulierten Thrombozyten herangezogen wird.

2. Verfahren nach Anspruch 1,
welches, insbesondere vollautomatisiert, in einem Analysesystem (1) zur optischen Analyse einer Probe (4) eines Biofluids durchgeführt wird.

3. Verfahren nach Anspruch 1 oder 2,
wobei die obere Grenze (10) des Teilbereiches des Brechungsindexes, insbesondere veränderlich über den Absorptionsgrad, derart gewählt wird, dass Brechungsindizes der Erythrozyten von Brechungsindizes der Thrombozyten separiert werden.

4. Verfahren nach Anspruch 3,
wobei die Häufigkeitsverteilung der gemessenen Brechungsindizes ermittelt wird, hierin eine Population (14) der Erythrozyten in einem Bereich höherer Brechungsindizes und eine Population (15) der Thrombozyten in einem Bereich niedrigerer Brechungsindizes identifiziert wird, und wobei als obere Grenze (10) des Teilbereiches des Brechungsindexes ein Brechungsindex aus einem Bereich zwischen den beiden Populationen (14, 15) gewählt wird.

5. Verfahren nach einem der vorhergehenden Ansprüche,
wobei die untere Grenze (11) des Teilbereiches des Absorptionsgrades, insbesondere veränderlich über den Absorptionsgrad, derart gewählt wird, dass Absorptionsgrade reifer Thrombozyten von Absorptionsgraden retikulierter Thrombozyten separiert werden.

6. Verfahren nach Anspruch 5,
wobei in der Probe (4) des Biofluids die Absorptionsgrade der Thrombozyten gemessen werden und deren Häufigkeitsverteilung ermittelt wird, wobei aus der Häufigkeitsverteilung ein Häufigkeitsmaximum bestimmt wird, und wobei als untere Grenze (11) des Teilbereiches des Absorptionsgrades ein Absorptionsgrad oberhalb des dem Häufigkeitsmaximum zugeordneten Absorptionsgrad gewählt wird.

7. Verfahren nach Anspruch 6,
wobei der Abstand der unteren Grenze (11) des Teilbereiches des Absorptionsgrades zu dem dem Häufigkeitsmaximum zugeordneten Absorptionsgrad derart gewählt wird, dass die für ein Normbiofluid aus den selektierten Messwerten ermittelte Fraktion der retikulierten Thrombozyten bezüglich aller Thrombozyten in einem Bereich zwischen 1% und 7% liegt.

8. Verfahren nach einem der vorhergehenden Ansprüche,
wobei die untere Grenze (12) des Teilbereiches des Brechungsindexes, insbesondere abhängig vom Absorptionsgrad, derart gewählt wird, dass Brechungsindizes der Thrombozyten von Brechungsindizes von Fragmenten der Leukozyten und anderen Teilchen separiert werden

9. Verfahren nach einem der vorhergehenden Ansprüche,
wobei in der Probe (4) des Biofluids, insbesondere automatisiert, eine Anzahl der Thrombozyten ermittelt wird, und wobei aus der Anzahl der selektierten Messwerte und der Anzahl der Thrombozyten eine Fraktion der retikulierten Thrombozyten ermittelt wird.

10. Analysesystem (1) zur optischen Analyse einer Probe (4) eines Biofluids, insbesondere einer Blutprobe, welches eingerichtet und ausgebildet ist, ein Verfahren mit den Merkmalen eines der vorhergehenden Ansprüche, insbesondere vollautomatisiert, durchzuführen.
